# EUROPEAN PATENT SPECIFICATION

(11) **EP 3 416 603 B1**
(45) Date of publication and mention of the grant of the patent: **11.12.2019**
(21) Application number: 17705456.6
(22) Date of filing: 08.02.2017
(51) Int. Cl.: A61G 7/057, A61B 5/00

(54) **SUPPORT EVALUATION DEVICE**
UNTERSTÜTZUNGSAUSWERTUNGSVORRICHTUNG
DISPOSITIF D'ÉVALUATION DE SUPPORT

(30) Priority: 17.02.2016 GB 201602771
(43) Date of publication of application: 26.12.2018
(73) Proprietor: The Helping Hand Company (Ledbury) Limited, Herefordshire HR8 1NS (GB)
(72) Inventor: JAMES, Gavin, Ledbury Herefordshire HR8 1NA (GB); MOORE, Stuart, Colwall Herefordshire WR13 6QP (GB)
(74) Representative: Games, Robert Harland
(86) International application number: PCT/GB2017/050310
(87) International publication number: WO 2017/141010

(56) References cited:
- DE-A1- 19 802 099
- US-A- 2 644 332

## Description

The present invention relates to a support evaluation device for use in prescribing a seat or cushion of optimal firmness to a person.

### BACKGROUND TO THE INVENTION

People who spend long periods of time sitting in a fixed position are at greater risk of developing pressure ulcers. Whilst the skin can tolerate a high pressure for short period, or sustained lower pressure over a longer period, spending excessive time in one position can cause capillaries in the skin to close up, leading to the subsequent formation of a pressure ulcer (also known as pressure sores or bedsores).

People that tend to be at higher risk include the elderly, and paraplegic, tetraplegic, critically ill or terminally ill patients in hospital, for example, because pressure ulcers increase the likelihood of infection for the immunocompromised. The most commonly affected parts of the body include the sacrum, the coccyx, the heels and the hips, although they can form elsewhere too.

Pressure ulcers typically form if there is sustained pressure on an area of soft tissue, often over a bony prominence. Various factors can affect this, including shearing (where the skin remains static but the underlying tissues do not) and the microclimate (temperature and level of moisture) between the bed and a patient's skin. Pressure ulcers are painful and tend to heal slowly, if at all, although this depends on the individual.

Some techniques for mitigating pressure ulcer development are known. Regularly turning bedridden patients redistributes pressure to other parts of the body, but this requires nursing staff, for example, to adhere to a regular schedule for a potentially large number of patients. Maintaining a healthy diet and effective management of the skin microclimate can each help combat pressure ulcers.

Seats and cushions have been optimised to provide better support than conventional seats during extended periods of use. Clinical staff typically use incrementally firmer seating for a patient on a reactive basis (i.e. trial and error), following postural assessment of a patient in various positions. For example, if pressure sores or their precursors are observed after a week of using a given seat, only then will a firmer seat be prescribed.

Some cushion-fitting clinics conduct an initial assessment using individual or multiple pressure sensors to take single or continuous readings of pressure at the skin surface. These readings can be recorded manually or via complex computer software for mapping skin pressure levels, a technique known as interface pressure mapping. However, it requires special training to use the software and interpret the results correctly. It is also time-consuming to set up the equipment to take the required measurements (which only measure pressure, not shear), and the results merely contribute to a clinician's overall assessment of the appropriate seat firmness required for a given patient, rather than giving a clear indication of a patient's requirements. A support evaluation device is known from US 2 644 332 A. DE 198 02 099 A1 discloses a pressure sensitive switch.

It is an objective of the present invention to reduce or substantially obviate the aforementioned problems.

### STATEMENT OF INVENTION

The invention is defined by the appended claims. According to a first aspect of the present invention, there is provided a support evaluation device comprising a series of pressure sensors in a stack, output means linked to the pressure sensors for indicating signals therefrom, and a power source,
the series of pressure sensors including at least first and second pressure sensors within the stack, operational across respective first and second pressure bands,
each pressure sensor including first and second electrical conductors, and an insulator spacing the electrical conductors,
the pressure sensors being adapted to signal whether there is contact between their electrical conductors, and
the output means being adapted to provide at least one of an audio or visual output corresponding to signals from the pressure sensors, in use indicating the pressure band reached for a patient sat on the device,
each pressure band corresponding to a predetermined cushion strength for prescribing a cushion to that patient.

Advantageously, the support evaluation device enables a carer, healthcare professional or system, for example, to prescribe a support cushion or seat with appropriate support to a person. This does not require trial and error, so it is not necessary to incrementally increase the strength of a support cushion/seat in reaction to the development of sores or pressure ulcers - the cushion/seat prescribed is correct from the outset. The device gives different outputs corresponding to different pressure bands (or ranges), for normal or excessively high/low pressure, for example. Each pressure sensor has a particular pressure threshold at which it activates, and that pressure threshold is the lower end value of one pressure band, and the higher value end of another pressure band. The absence of a signal may be used to indicate a state of the pressure sensor too.

In use, a person sits on the evaluation device and compresses one or more of the insulators in the stack, for example. This brings one or more of the electrical conductors into contact, activating the respective pressure sensor and completing the circuit in respect of those conductors, leading to activation of the output means. The output means gives an output corresponding to the particular conductors which are in contact, indicating to the carer which pressure band has been reached (or energised). This result is then used to prescribe a support cushion or other seat having appropriate characteristics or properties (or to calibrate such a cushion or seat, where possible).

The device is also particularly advantageous because it senses pressure across a continuous area, rather than at discrete measurement points. This allows high pressure to be detected on any seated parts of the person using the device. Overall, the support evaluation device functions as a diagnostic tool for clinical assessment of a seated person, resulting in improved patient care by reducing diagnostic uncertainty in prescribing support cushions or associated seating.

A third pressure sensor may also be provided in the series of pressure sensors in the stack.

Providing a third pressure sensor enables the device to monitor pressure across four different pressure bands as opposed to three, the first being the band where no pressure sensors are triggered. This can enable readings across a wider range of pressures (for example, a broader range of user weights), if the existing pressure bands are kept unaltered. Alternatively, this can allow narrower pressure bands to be used across the same pressure range, making the device more sensitive to pressure within the existing pressure range.

At least one of the pressure sensors may share a common electrical conductor with another of the pressure sensors.

Using a common or shared electrical conductor (i.e. the second electrical conductor of the one pressure sensor is the first electrical conductor of another pressure sensor) can make the device more sensitive to pressure, reducing the difference between pressure thresholds corresponding to the different pressure bands. This avoids the inclusion of additional intermediate layers which would shift the pressure bands being measured.

The series of pressure sensors may be concatenated in the stack. The electrical conductors and insulators may be provided in alternating strata or layers.

By concatenating or interconnecting the pressure sensors, the operation of a given pressure sensor is dependent on the operation of the preceding pressure sensors in the stack. Therefore, pressure sensors (corresponding to higher pressure bands) can only be activated if preceding pressure sensors (at lower pressure bands) have already been activated, preventing the output means erroneously indicating pressure in a higher band than the pressure being applied.

Providing the conductors and insulators in alternating layers improves operation of the pressure sensors in series, as opposed to having separate electrical conductors for each pressure sensor. Using alternating layers also allows the pressure bands to be customised according to the thickness and firmness of the insulator used in each layer.

The uppermost pressure sensor in the stack may operate above a first pressure threshold. Each subsequent pressure sensor in the stack may operate above a successively higher pressure threshold.

By having pressure sensors which operate above various pressure thresholds, activation of a given pressure sensor corresponds to applied pressure falling within a particular pressure band. This indication can be used to calibrate or prescribe an appropriately supportive cushion for a person for longer term use.

The insulators may be made of resilient foam. The insulators may be made of combustion modified high resilience (CMHR) foam.

Advantageously, this ensures consistent readings are taken when a person sits on the device. CMHR foam also meets relevant Fire Safety Regulations, having enhanced fire resistance properties, and resists bacterial/fungal growth.

Each insulator may include one or more apertures through which the electrical conductors can come into contact with each other.

The pressure sensors operate when pressure applied to the device compresses one electrical conductor into another through the apertures. This allows each pressure sensor to function as a switch for a predetermined pressure threshold, when that threshold is exceeded for a given layer of the stack. The apertures are dispersed around each insulating layer allowing each pressure sensor functions across the whole of the device, which avoids the need to quantify the pressure applied to a particular area in use.

The insulator in the uppermost pressure sensor of the stack may be thinner than the insulators in the subsequent pressure sensors.

This enables to uppermost pressure sensor to operate across a pressure band corresponding to comparatively low pressure on the device, where a young child is being assessed, for example. In other words, the pressure threshold for activating the first pressure sensor is low. For pressure sensors in series, using a thinner insulator for the first pressure sensor also allows some modulation of the pressure bands across which subsequent pressure sensors in the series are operational, without changing those layers directly.

Each electrical conductor may include a carbon-printed surface. Each electrical conductor may include carbon-printed polythene. The electrical conductors may include a fabric or other material which can flow (or slip) into the apertures in the insulator.

It is safer to use electrical conductors with carbon-printed surfaces, as opposed to metal for example, because this prevents electrical arcing between conductors if they are proximal but not in contact. Carbon-printed surfaces are also more durable than metal counterparts over repeated cycles of compression. Carbon-printed polythene is particularly durable and flexible.

The output means may include a speaker for providing audible output. The speaker may provide a tone-based and/or verbal output. The output means may include one or more LEDs for providing visual output.

Either or both of these outputs can be used to provide simple and quickly determinable results when using the support evaluation device, without requiring intensive training. Illuminating one, two or three LEDs, for example, can indicate that a soft, medium or hard cushion may be appropriate for prolonged use by a given person. Sequences of beeps/tones can signal whether pressure is too low/high, or within an acceptable range. Verbal indications can accomplish the same without requiring the user to interpret the meaning of beeps/tones.

The output means may include a signal for communication with an external device. The signal may be transmitted wirelessly for receipt by the external device.

The signal can send diagnostic information from the support evaluation device to the external device (e.g. a computer or computer system), so that the results of the support assessment are recorded automatically. The signal may be sent to multiple external devices. A wireless signal is useful to streamline use of the device, avoiding the need to connect cables prior to use.

The support evaluation device may be incorporated into a cushion or a seat.

According to a second aspect of the present invention, there is provided a kit comprising a support evaluation device according to the first aspect of the invention, and at least first and second pressure monitoring cushions, the first cushion having a predetermined cushion strength corresponding to the first pressure band of the support evaluation device, and the second cushion having a predetermined cushion strength corresponding to the second pressure band of the support evaluation device.

Following use of the support evaluation device, a patient can be provided with a cushion of lesser or greater firmness, depending on the pressure band determined by the support evaluation device. This ensures that the patient can be prescribed a cushion suitably adapted for their needs, rather than providing a stock cushion of inadequate strength to provide proper support for that patient.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows an exploded perspective view of a first embodiment of a support evaluation device;
Figure 2 shows a partial side cross-sectional view through the device of Figure 1;
Figure 3 shows a side cross-sectional view of the device of Figure 1 in use, with a person seated atop the device; and
Figure 4 shows a side cross-sectional view of a second embodiment, not belonging to the invention, of a support evaluation device in use, with a person seated atop the device.

### DESCRIPTION OF PREFERRED EMBODIMENTS

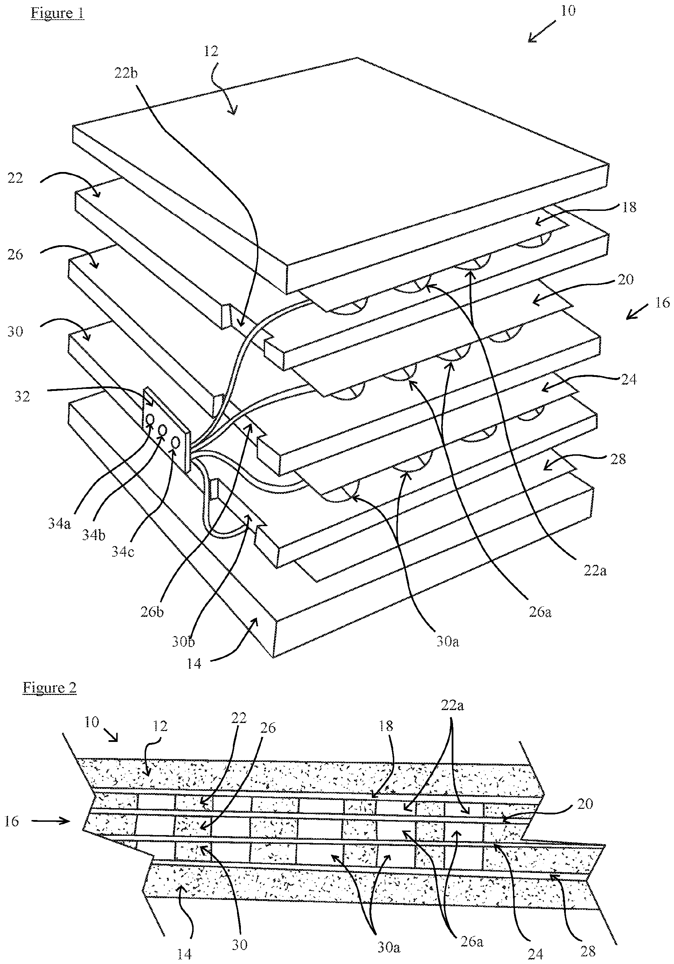

Referring firstly to Figure 1, a first embodiment of a support evaluation device is indicated generally at 10. The device 10 is a cushion including a top cushion 12 and a bottom cushion 14. Each cushion 12, 14 includes a sheet of foam. The foam is resilient and can be compressed. A stack 16 of pressure sensors is disposed between the cushions 12, 14. The cushions 12, 14 are each 10 mm thick in this embodiment. Each pressure sensor has an area of approximately the same size and shape. The pressure sensors are disposed coaxially through the stack 16.

The stack 16 includes a first pressure sensor consisting of electrically conductive sheets (or layers) 18, 20 on either side of a foam layer (or sheet) 22. In this embodiment, the foam layer in the first pressure sensor is 5 mm thick. The stack 16 also includes a second pressure sensor consisting of electrically conductive sheets 20, 24 on either side of a secondary foam layer 26. The stack 16 also includes a third pressure sensor consisting of electrically conductive sheets 24, 28 on either side of a tertiary foam layer 30. The secondary and tertiary foam layers are each 10 mm thick in this embodiment. The foam layers 22, 26, 30 are electrically insulating. Each foam layer 22, 26, 30 is resilient and compressible.

The pressure sensors are arranged in layers or tiers within the stack 16. The layers are substantially parallel. Each pressure sensor lies in a different horizontal plane of the stack. In other words, the pressure sensors lie on top of one another. The conductive sheets 18, 20, 24, 28 and foam layers 22, 26, 30 alternate within the stack, concatenating the pressure sensors. Each pressure sensor functions as a pressure switch. Higher pressures are needed to operate pressure sensors disposed lower down in the stack. This is because more material must be deformed to bring the corresponding conductive sheets into contact.

The foam layers 22, 26, 30 and cushions 12, 14 in this embodiment are all 50/230 grade foam. Additionally, the foam layers 22, 26, 30 and cushions 12, 14 are combustion modified high resilience (CMHR) foam. Each foam layer 22, 26, 30 is a spacer between the conductive sheets 18, 20, 24, 28. The foam layers 22, 26, 30 each include a plurality of apertures or holes 22a, 26a, 30a. The axes of each aperture 22a, 26a, 30a runs approximately in line with the length of the stack 16. The apertures 22a, 26a, 30a are arranged in rows extending from one side to the other of the respective foam layers 22, 26, 30. The apertures 22a, 26a, 30a may be arranged regularly or irregularly in other embodiments.

The apertures 22a, 26a, 30a in each foam layer 22, 26, 30 are co-aligned. The foam layers 22, 26, 30 are compressed when the device 10 is sat upon, but the apertures 22a, 26a, 30a substantially retain their size, shape and relative spacing. The conductive sheets 18, 20, 24, 28 cover the apertures 22a, 26a, 30a on the sides of the respective foam layers 22, 26, 30. In other embodiments, the apertures in different foam layers may be anti-aligned, or have another alignment.

The conductive sheets 18, 20, 24, 28 are each carbon-printed (or carbon-coated) sheets of polythene. Polythene is durable and can undergo deformation through the relevant apertures in the foam layers 22, 26, 30 over many cycles without wearing out. Using carbon as a conductor is better than metal because it is lighter, it will not undergo metal fatigue during repeated compression cycles. Additionally, internal arcing across air gaps will not occur if a small gap is present between a pair of conductive sheets.

The first, second and third pressure sensors function in series within the stack. The first pressure sensor is uppermost in the stack 16. The first and second pressure sensors are adjacent and share a common conductive sheet 20. Operation of the second pressure sensor is conditional on operation of the first pressure sensor. The second and third pressure sensors are adjacent and also share a common conductive sheet 24. The common conductive sheets each form an interface between respective foam layers. Operation of the third pressure sensor is contingent on operation of the first and second pressure sensors. Note that other embodiments may instead have distinct conductive sheets for each pressure sensor, which may be separated by an insulating layer.

By using three pressure sensors, pressure falling within four different pressure bands can be measured or detected using the device 10. At an applied pressure of less than around 80 mmHg, none of the pressure sensors are brought into operation. At a pressure threshold of around 80 mmHg, the first pressure sensor is activated. At a second pressure threshold of around 130 mmHg. At a third pressure threshold of around 170 mmHg, the third pressure sensor is activated. At an applied pressure above around 170 mmHg, all three of the pressure sensors are brought into operation.

From the above pressure thresholds, the four pressure bands correspond to pressure in the following ranges: less than around 80 mmHg, between around 80-130 mmHg, between around 130-170 mmHg, and above around 170 mmHg. Support cushions may be provided to provide incrementally firmer support corresponding to the successively higher pressure bands.

Each of the pressure sensors can be brought into operation under sufficient pressure. This is because, in use, the foam layers compress into a significantly smaller volume where the applied pressure is highest, shortening the distance the conductive sheets must bridge to make electrical contact through the foam layer. This allows detection of the highest pressure at any single point on the device 10, and hence any part of the person seated on the device. In other words, the device 10 provides a continuous sensing region or area, rather than being limited to individual sensor inputs located in discrete areas.

An output assembly 32 is provided at the side of the device 10. The output assembly is connected to each of the conductive sheets 18, 20,24, 28 by electrical wires. The output assembly 32 includes three LEDs 34a, 34b, 34c. The output assembly also includes a battery for powering the LEDs and pressure sensors. The output assembly includes a controller (not shown) programmed to control the LEDs 34a, 34b, 34c according to input signals received from the pressure sensors. Together, the controller, the conductive sheets 18, 20, 24, 28, the LEDs 34a, 34b, 34c and the battery form parts of an electrical circuit.

In this embodiment, the controller controls the LEDs in the following manner. The first LED 34a is lit when the conductive sheets 18, 20 of the first pressure sensor connect. The second LED 34b is lit when the conductive sheets 20, 24 of the second pressure sensor connect. The third LED 34c is lit when the conductive sheets 24, 28 of the third pressure sensor connect. In alternative embodiments, the LEDs may flash or light concurrently, for example, depending on the pressure applied to the device 10 in use. It will be appreciated that the LEDs could be normally 'on', and individually deactivate to signal connection of the respective conductive sheets, for example.

In this embodiment, the LEDs 34a, 34b, 34c are green, amber and red. The green LED indicates the first pressure sensor is in operation. The amber LED indicates the second pressure sensor is in operation. The red LED indicates the third pressure sensor is in operation. This provides a visual cue regarding the strength of support cushion to be prescribed for a patient.

The foam layers 22, 26, 30 also each include a side cut-out or recess 22b, 26b, 30b. When assembled, the output assembly 32 fits into these recesses 22b, 26b, 30b so that it does not protrude from the side of the device 10.

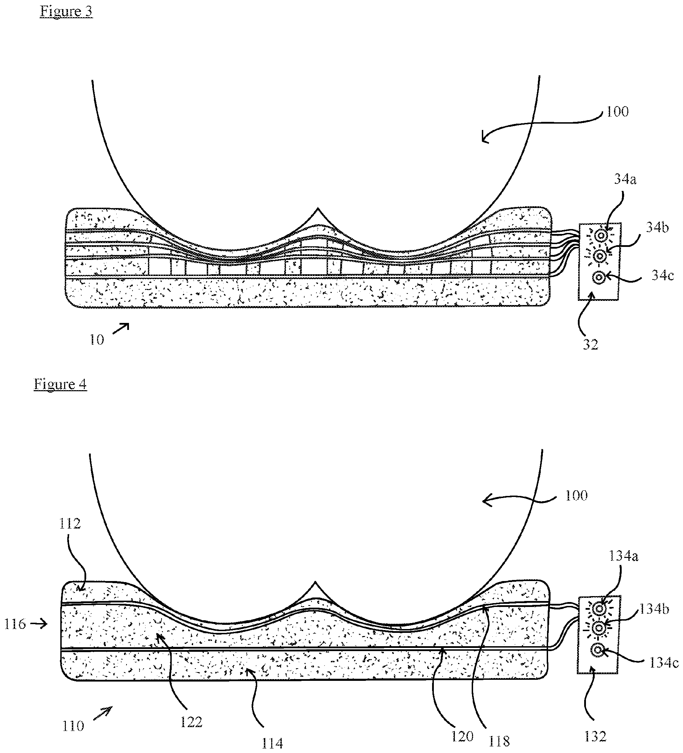

Referring now to Figures 2 and 3, the device can be seen before and during use by a person 100 respectively. It can be seen that the foam layers 22, 26 in the first and second pressure sensors are substantially collapsed beneath the person 100 in Figure 3, and the respective conductive sheets 18, 20, 24 come into contact. This signals the controller to light the LEDs 34a, 34b corresponding to those pressure sensors. The foam layer 30 in the third pressure sensor is not collapsed, and so the corresponding LED 34c is not lit. The device 10 therefore indicates that a cushion corresponding to support for pressure in the range 130-170 mmHg is appropriate for the person 100.

In use, it is envisaged that the device 10 will be used as part of a clinical assessment of a patient who spends significant amounts of time sitting or lying down. The device 10 may be provided integrated into (i.e. built into) a cushion of a chair, for example, and may be provided with a removable washable cover.

When a patient is sat atop the device 10, the pressure exerted on the device causes the uppermost conductive sheet 18 to enter one or more of the apertures 22a in the uppermost foam layer 22. Over time, depending on the pressure applied, other conductive sheets may also come into contact in either the second or the second and third pressure sensors beneath the first pressure sensor. This activates the LEDs 34a, 34b, 34c corresponding to the operational/active pressure sensors. In turn, this enables prescription of a cushion capable of providing support appropriate to the highest pressure band measured by the pressure sensors.

Referring now to Figure 4, a second embodiment, not belonging to the invention, of the support evaluation device is indicated generally at 110. The device 110 is a cushion and includes a top cushioned layer 112 and a bottom cushioned layer 114. Each layer 112, 114 includes a sheet of foam which is compressible and resilient. A pressure sensor layer 116 is disposed between the cushioned layers 112, 114. The cushioned layers 112, 114 are each 10 mm thick in this embodiment.

The pressure sensor includes two electrically conductive sheets (or layers) 118, 120 on either side of a foam layer (or sheet) 122. The foam layer is electrically insulating, resilient and compressible. The other features of the sensor 116 are similar to those of the first embodiment, except that apertures are not provided through the insulating foam layer 122.

An output assembly 132 is provided at the side of the device 110. The output assembly is connected to each of the conductive sheets 118, 120 by electrical wires. The output assembly 132 includes three LEDs 134a, 134b, 134c. The output assembly 132 also includes a battery (not shown) for powering the LEDs 134a, 134b, 134c and pressure sensor 116. The output assembly 132 further includes a controller (not shown) programmed to control the LEDs 134a, 134b, 134c according to input signals received from the pressure sensor 116.

The controller is programmed to measure the capacitance of the pressure sensor 116. When the person 100 has sat on the cushion, depressions corresponding to the ischial tuberosities are formed in the device 110. This causes the capacitance of the cushion to change. The controller measures and records the initial (uncompressed) and final (compressed) values as signals from the pressure sensor 116.

The controller can determine the pressure being exerted on the cushion 110 based on the capacitance change, relative to its initial value. This also works if the initial state is another compressed state. This information is used to light up the appropriate LED (or LEDs) 134a, 134b, 134c to indicate the firmness of another cushion to be prescribed. A speaker may be used in another embodiment of the capacitive support evaluation device.

Other embodiments are envisaged within the scope of the claims. For example, a similar cushion to the first embodiment may be provided with capacitive pressure sensors, without apertures through the insulators, and the controller is adapted to measure the capacitance of each sensor. Alternatively, there may not be a controller for the output means, if contact between the conductive sheets is used to directly initiate output. Wires in the capacitive version may be connected across a plurality of points in the or each pressure sensor, allowing a series of capacitance/pressure measurements to be taken to distinguish regions of different pressure for different parts of the body. This could be an alternative or additional feature to having a plurality of sensors operating in parallel.

One or more speakers may be provided instead of or in addition to the LEDs. The speakers could output different tones to indicate the pressure bands measured during use. A signal (possibly wireless) could be sent to an external system, such as a hospital records system, to record the results of a patient using the evaluation device for future reference. The device may be incorporated into a mattress or bed, for evaluating support across the whole of a person's body. A combination of pressure sensors, some have apertures through the insulators and some being capacitive, may be provided. The weight of the person sitting on the device may be a power source for powering the output, for example.

The embodiments described above are provided by way of example only, and various changes and modifications will be apparent to persons skilled in the art without departing from the scope of the present invention as defined by the appended claims.

## Claims

1. A clinical support evaluation device (10) comprising a series of pressure sensors in a stack (16), output means (32) linked to the pressure sensors for indicating signals therefrom, and a power source,
the series of pressure sensors including at least first and second pressure sensors within the stack (16), operational across respective first and second pressure bands,
each pressure sensor including first and second sheets of electrical conductors (18, 20, 24, 28), and a collapsible foam insulator (22, 26, 30) spacing the electrical conductors (18, 20, 24, 28), the insulator (22, 26, 30) including one or more apertures (22a, 26a, 30a) through which the sheets of electrical conductors (18, 20, 24, 28) can come into contact when the foam (22, 26, 30) is collapsed during use,
the pressure sensors being adapted to signal whether there is contact between their electrical conductors (18, 20, 24, 28), and
the output means (32) being adapted to activate when the electrical conductors (18, 20, 24, 28) of the pressure sensor(s) are in contact, and provide at least one of an audio or visual output corresponding to signals from the pressure sensors, in use indicating the pressure band reached for a patient sat on the device (10),
each pressure band corresponding to a predetermined cushion strength for prescribing a cushion to that patient.

2. A clinical support evaluation device (10) as claimed in claim 1, in which a third pressure sensor is also provided in the series of pressure sensors in the stack (16).

3. A clinical support evaluation device (10) as claimed in claim 1 or claim 2, in which at least one of the pressure sensors shares a common electrical conductor (20, 24) with another of the pressure sensors.

4. A clinical support evaluation device (10) as claimed in any of claims 1 to 3, in which the series of pressure sensors is concatenated in the stack (16), the electrical conductors and insulators being provided in alternating strata.

5. A clinical support evaluation device (10) as claimed in any preceding claim, in which the uppermost pressure sensor in the stack (16) operates above a first pressure threshold, and each subsequent pressure sensor in the stack (16) operates above a successively higher pressure threshold.

6. A clinical support evaluation device (10) as claimed in any preceding claim, in which the insulators (22, 26, 30) are resilient and made of foam.

7. A clinical support evaluation device (10) as claimed in any preceding claim, in which the insulators (22, 26, 30) are made of combustion modified high resilience (CMHR) foam.

8. A clinical support evaluation device (10) as claimed in any preceding claim, in which the insulator (22) in the uppermost pressure sensor of the stack (16) is thinner than the insulators (26, 30) in the subsequent pressure sensors.

9. A clinical support evaluation device (10) as claimed in any preceding claim, in which each electrical conductor (18, 20, 24, 28) includes a carbon-printed surface.

10. A clinical support evaluation device (10) as claimed in any preceding claim, in which each electrical conductor (18, 20, 24, 28) includes carbon-printed polythene.

11. A clinical support evaluation device (10) as claimed in any preceding claim, in which the output means (32) includes a speaker for providing audible output.

12. A clinical support evaluation device (10) as claimed in any preceding claim, in which the output means (32) includes one or more LEDs (34a, 34b, 34c) for providing visual output.

13. A clinical support evaluation device (10) as claimed in any preceding claim, in which the output means (32) includes a signal for communication with an external device.

14. A clinical support evaluation device (10) as claimed in any preceding claim, incorporated into a cushion or a seat.

15. A kit comprising a clinical support evaluation device (10) as claimed in any of claims 1 to 14, and at least first and second pressure monitoring cushions, the first cushion having a predetermined cushion strength corresponding to the first pressure band of the clinical support evaluation device (10), and the second cushion having a predetermined cushion strength corresponding to the second pressure band of the clinical support evaluation device (10).

## Patentansprüche

1. Klinische Stützbewertungsvorrichtung (10), die eine Reihe von Drucksensoren in einem Stapel (16), Ausgabemittel (32), die mit den Drucksensoren verbunden sind, um Signale aus denselben anzuzeigen, und eine Energiequelle umfasst,
wobei die Reihe von Drucksensoren mindestens erste und zweite Drucksensoren innerhalb des Stapels (16) einschließt, die über jeweilige erste und zweite Druckbänder arbeiten,
wobei jeder Drucksensor erste und zweite Lagen aus elektrischen Leitern (18, 20, 24, 28) und eine zusammendrückbaren Schaumstoffisolierung (22, 26, 30) einschließt, die die elektrischen Leiter (18, 20, 24, 28) beabstandet, wobei die Isolierung (22, 26, 30) eine oder mehrere Öffnungen (22a, 26a, 30a) einschließt, durch die die Lagen aus elektrischen Leitern (18, 20, 24, 28) in Kontakt kommen können, wenn der Schaumstoff (22, 26, 30) im Gebrauch zusammengedrückt wird,
wobei die Drucksensoren dazu geeignet sind, zu signalisieren, ob Kontakt zwischen ihren elektrischen Leitern (18, 20, 24, 28) besteht, und
wobei die Ausgabemittel (32) dazu geeignet sind, sich zu aktivieren, wenn sich die elektrischen Leiter (18, 20, 24, 28) des/der Drucksensor(en) in Kontakt befinden, und mindestens eines aus einer Audio- oder visuellen Ausgabe bereitzustellen, die Signalen aus den Drucksensoren entspricht, welche im Gebrauch das Druckband anzeigen, das bei einem Patienten erreicht wurde, der auf der Vorrichtung (10) saß,
wobei jedes Druckband einer vorbestimmten Polsterstärke zum Verschreiben eines Polsters für diesen Patienten entspricht.

2. Klinische Stützbewertungsvorrichtung (10) nach Anspruch 1, wobei in der Reihe von Drucksensoren im Stapel (16) ebenfalls ein dritter Drucksensor bereitgestellt ist.

3. Klinische Stützbewertungsvorrichtung (10) nach Anspruch 1 oder Anspruch 2, wobei mindestens einer der Drucksensoren einen gemeinsamen elektrischen Leiter (20, 24) mit einem anderen der Drucksensoren teilt.

4. Klinische Stützbewertungsvorrichtung (10) nach einem der Ansprüche 1 bis 3, wobei die Reihe von Drucksensoren im Stapel (16) verkettet ist, wobei die elektrischen Leiter und Isolatoren in abwechselnden Schichten bereitgestellt sind.

5. Klinische Stützbewertungsvorrichtung (10) nach einem vorstehenden Anspruch, wobei der oberste Drucksensor im Stapel (16) über einer ersten Druckschwelle arbeitet, und jeder nachfolgende Drucksensor im Stapel (16) über einer sukzessive höheren Druckschwelle arbeitet.

6. Klinische Stützbewertungsvorrichtung (10) nach einem vorstehenden Anspruch, wobei die Isolierungen (22, 26, 30) elastisch und aus Schaumstoff gefertigt sind.

7. Klinische Stützbewertungsvorrichtung (10) nach einem vorstehenden Anspruch, wobei die Isolierungen (22, 26, 30) verbrennungsmodifizierten hochelastischen Schaumstoff (CMHR, abgekürzt aus dem Englischen für *combustion modified high resilience foam*) gefertigt sind.

8. Klinische Stützbewertungsvorrichtung (10) nach einem vorstehenden Anspruch, wobei die Isolierung (22) im obersten Drucksensor des Stapels (16) dünner ist als die Isolierungen (26, 30) in den nachfolgenden Drucksensoren.

9. Klinische Stützbewertungsvorrichtung (10) nach einem vorstehenden Anspruch, wobei jeder elektrische Leiter (18, 20, 24, 28) eine kohlenstoffbedruckte Fläche einschließt.

10. Klinische Stützbewertungsvorrichtung (10) nach einem vorstehenden Anspruch, wobei jeder elektrische Leiter (18, 20, 24, 28) kohlenstoffbedrucktes Polyethylen einschließt.

11. Klinische Stützbewertungsvorrichtung (10) nach einem vorstehenden Anspruch, wobei das Ausgabemittel (32) einen Lautsprecher zum Bereitstellen von hörbarer Ausgabe einschließt.

12. Klinische Stützbewertungsvorrichtung (10) nach einem vorstehenden Anspruch, wobei das Ausgabemittel (32) eine oder mehrere LEDs (34a, 34b, 34c) zum Bereitstellen von visueller Ausgabe einschließt.

13. Klinische Stützbewertungsvorrichtung (10) nach einem vorstehenden Anspruch, wobei das Ausgabemittel (32) ein Signal zur Kommunikation mit einer externen Vorrichtung einschließt.

14. Klinische Stützbewertungsvorrichtung (10) nach einem vorstehenden Anspruch, die in einem Polster oder einem Sitz integriert ist.

15. Set, das eine klinische Stützbewertungsvorrichtung (10) nach einem der Ansprüche 1 bis 14, und mindestens erste und zweite Drucküberwachungspolster umfasst, wobei das erste Polster eine vorbestimmte Polsterfestigkeit aufweist, die dem ersten Druckband der klinischen Stützbewertungsvorrichtung (10) entspricht, und das zweite Polster eine vorbestimmte Polsterfestigkeit aufweist, die dem zweiten Druckband der klinischen Stützbewertungsvorrichtung (10) entspricht.

## Revendications

1. Dispositif d'évaluation de support clinique (10) comprenant une série de capteurs de pression dans un empilement (16), des moyens de sortie (32) reliés aux capteurs de pression pour indiquer des signaux issus de ceux-ci, et une source d'alimentation,
la série de capteurs de pression incluant au moins un premier et un deuxième capteur de pression au sein de l'empilement (16), opérationnels respectivement sur une première et une deuxième bande de pression,
chaque capteur de pression incluant des première et deuxième feuilles de conducteurs électriques (18, 20, 24, 28), et un isolant en mousse compressible (22, 26, 30) espaçant les conducteurs électriques (18, 20, 24, 28), l'isolant (22, 26, 30) incluant une ou plusieurs ouvertures (22a, 26a, 30a) à travers lesquelles les feuilles de conducteurs électriques (18, 20, 24, 28) peuvent venir en contact lorsque la mousse (22, 26, 30) est comprimée pendant une utilisation,
les capteurs de pression étant adaptés pour signaler s'il y a un contact entre leurs conducteurs électriques (18, 20, 24, 28), et
les moyens de sortie (32) étant adaptés pour s'activer lorsque les conducteurs électriques (18, 20, 24, 28) du (des) capteur(s) de pression sont en contact, et fournir au moins l'une d'une sortie audio ou visuelle correspondant à des signaux provenant des capteurs de pression, indiquant pendant l'utilisation la bande de pression atteinte pour un patient assis sur le dispositif (10),
chaque bande de pression correspondant à une résistance de coussin prédéterminée pour prescrire un coussin à ce patient.

2. Dispositif d'évaluation de support clinique (10) selon la revendication 1, dans lequel un troisième capteur de pression est également prévu dans la série de capteurs de pression dans l'empilement (16).

3. Dispositif d'évaluation de support clinique (10) selon la revendication 1 ou la revendication 2, dans lequel au moins l'un des capteurs de pression partage un conducteur électrique commun (20, 24) avec un autre des capteurs de pression.

4. Dispositif d'évaluation de support clinique (10) selon l'une quelconque des revendications 1 à 3, dans lequel la série de capteurs de pression est concaténée dans l'empilement (16), les conducteurs électriques et isolants étant ménagés en strates alternées.

5. Dispositif d'évaluation de support clinique (10) selon une quelconque revendication précédente, dans lequel le capteur de pression le plus haut dans l'empilement (16) fonctionne au-dessus d'un premier seuil de pression, et chaque capteur de pression ultérieur dans l'empilement (16) fonctionne au-dessus d'un seuil de pression successivement plus haut.

6. Dispositif d'évaluation de support clinique (10) selon une quelconque revendication précédente, dans lequel les isolants (22, 26, 30) sont résilients et constitués de mousse.

7. Dispositif d'évaluation de support clinique (10) selon une quelconque revendication précédente, dans lequel les isolants (22, 26, 30) sont constitués d'une mousse haute résilience modifiée par combustion (CMHR, abrégé de l'anglais *combustion modified high resilience foam*).

8. Dispositif d'évaluation de support clinique (10) selon une quelconque revendication précédente, dans lequel l'isolant (22) dans le capteur de pression le plus haut de l'empilement (16) est plus fin que les isolants (26, 30) dans les capteurs de pression ultérieurs.

9. Dispositif d'évaluation de support clinique (10) selon une quelconque revendication précédente, dans lequel chaque conducteur électrique (18, 20, 24, 28) inclut une surface imprimée au carbone.

10. Dispositif d'évaluation de support clinique (10) selon une quelconque revendication précédente, dans lequel chaque conducteur électrique (18, 20, 24, 28) inclut du polythène imprimé au carbone.

11. Dispositif d'évaluation de support clinique (10) selon une quelconque revendication précédente, dans lequel les moyens de sortie (32) incluent un haut-parleur destiné à fournir une sortie audible.

12. Dispositif d'évaluation de support clinique (10) selon une quelconque revendication précédente, dans lequel les moyens de sortie (32) incluent une ou plusieurs DEL (34a, 34b, 34c) destinées à fournir une sortie visuelle.

13. Dispositif d'évaluation de support clinique (10) selon une quelconque revendication précédente, dans lequel les moyens de sortie (32) incluent un signal pour communication avec un dispositif externe.

14. Dispositif d'évaluation de support clinique (10) selon une quelconque revendication précédente, incorporé dans un coussin ou un siège.

15. Nécessaire comprenant un dispositif d'évaluation de support clinique (10) tel que revendiqué dans l'une quelconque des revendications 1 à 14, et au moins un premier et un deuxième coussin de surveillance de pression, le premier coussin ayant une première résistance de coussin prédéterminée correspondant à la première bande de pression du dispositif d'évaluation de support clinique (10), et le deuxième coussin ayant une résistance de coussin prédéterminée correspondant à la deuxième bande de pression du dispositif d'évaluation de support clinique (10).
